# EUROPEAN PATENT SPECIFICATION

(11) **EP 2 123 118 B1**
(45) Date of publication and mention of the grant of the patent: **25.12.2013**
(21) Application number: 08719393.4
(22) Date of filing: 12.03.2008
(51) Int. Cl.: H05B 1/02, A61F 7/02

(54) **DEVICE AND METHOD FOR TEMPERATURE MANAGEMENT OF HEATING PAD SYSTEMS**
EINRICHTUNG UND VERFAHREN ZUR TEMPERATURVERWALTUNG VON HEIZPADSYSTEMEN
DISPOSITIF ET PROCÉDÉ DE GESTION DE TEMPÉRATURE DE SYSTÈMES DE MATELAS CHAUFFANTS

(30) Priority: 12.03.2007 US 894438 P
(43) Date of publication of application: 25.11.2009
(60) Divisional application: 13151084.4
(73) Proprietor: Medline Industries, Inc., Mundelein, IL 60060 (US)
(72) Inventor: ELLIS, Kent, D., Seattle, WA 98144 (US)
(74) Representative: Jacob, Reuben Ellis
(86) International application number: PCT/IB2008/000771
(87) International publication number: WO 2008/110922

(56) References cited:
- WO-A-01/95841
- WO-A-2006/076148
- WO-A-2006/086513
- US-A- 5 948 303
- US-A1- 2001 020 303
- US-A1- 2002 156 509
- US-A1- 2003 013 998
- US-A1- 2003 218 003

## Description

### BACKGROUND OF THE INVENTION

### Field of the Invention

This invention is in the field of heating control circuits for thermal pads and more particularly to temperature management of thermal pad systems.

### Description of the Related Art

Maintaining the temperature of the patient has recently been realized as a more important factor in the providing for the health than previously thought. The temperature of the patient may drop during surgery, when anesthesia is administered causing sleep, during shock, or as a result of a number of medical conditions. In such situations, providing a blanket to keep the warmth in the patient will often be insufficient to maintain and control the patient to the right temperature.

Some current systems that provide a flow of heated air over the patient may not provide sufficient control of the proper temperature of the patient. It may also be hard to ensure that the heat of the air is properly transferred to the patient, while at the same time, not overheating any doctors or medical personal who are performing medical procedures on the patient. In addition, Intravenous fluid delivery, such as drug delivery, is common in many general medical and surgery situations. There are no systems today that integrate into a single power supply and heating system the devices that will maintain the proper temperature of all sources of heat the patient, such as from an IV fluid, a thermal pad, room heat, in conjunction with monitoring and controlling the current temperature of the patient's body.

A thermal pad, as disclosed in U.S. Patent Nos. 6,653,607, 6,924,467, 6,933,469 and 6,967,309, heats to maintain patient normal-thermia with a unique X-ray transparent heating element. Temperature of the heating element is control by fiber optic sensor feed back signal (Photon Controls). This signal is the highest temperature recorded at various locations on the surface cover of the pad. Safe control of thermals applied to a patient's skin is the primary function of this control design, but this is not sufficient to ensure that the patient's body is at the correct temperature.

The thermal pad disclosed in the abovementioned U.S. patents fails to provide a means to prevent the surface temperature of the thermal pad from exceeding a safe temperature for warming a patient. In other words, in the event that a patient having a substantially large mass is to be heated while on the thermal pad, the body mass would cause a substantial thermodynamic transfer of heat from the patient to the thermal pad. The patient's body would heat the surface temperature of the thermal pad above a selected safe temperature of the thermal pad for warming the patient at the very start of the procedure, but then not be able to respond appropriately to the changes in temperature needed throughout the surgery. As such, the thermal pad design of the above U.S. patents fails to guarantee that the surface temperature of the thermal pad will not exceed the selected safe temperature of the thermal pad. Document US-A-5 948 303 discloses a temperature control for a bed according to the preamble of claim 1.

It is therefore desirable to have a thermal pad that addresses or alleviates at least some of the above stated problems.

### BRIEF SUMMARY OF THE INVENTION

According to one embodiment, an apparatus for warming a patient on a thermal pad includes a heating element to heat a surface of the thermal pad, a power unit operable to provide power to the heating element, a plurality of surface temperature sensors coupled to detect temperature at select portions of the surface of the thermal pad, a heating element temperature sensor coupled to detect a temperature of the heating element, and a temperature control circuit board embedded within the thermal pad and electrically coupled to the plurality of sensors and the heating element sensor, the temperature control circuit board operable to maintain a temperature at the surface of the thermal pad to a safe temperature based on the temperature detected by the plurality of sensors and the heating element sensor.

According to another embodiment, a method for warming a patient on a thermal pad includes providing a heating element to heat a surface of the thermal pad, providing a power unit to power the heating element, coupling a plurality of sensors proximate select portions of the surface of the thermal pad to detect temperature at the select portions of the surface, coupling a heating element proximate the heating element to detect a temperature of the heating element, and regulating the power provided to the heating element based on the temperatures detected by the plurality of sensors and the heating element sensor to limit a temperature at the surface of the thermal pad to a safe temperature.

### BRIEF DESCRIPTION OF THE SEVERAL VIEWS OF THE DRAWING(S)

In the drawings, identical reference numbers identify similar elements or acts. The sizes and relative positions of elements in the drawings are not necessarily drawn to scale. For example, the shapes of various elements and angles are not drawn to scale, and some of these elements are arbitrarily enlarged and positioned to improve drawing legibility. Further, the particular shapes of the elements as drawn, are not intended to convey any information regarding the actual shape of the particular elements, and have been solely selected for ease of recognition in the drawings.
Figure 1 is a schematic illustration of a temperature management system, according to one embodiment of the invention.
Figure 2A is an isometric exploded view of the thermal pad having a temperature control circuit board embedded therein, according to one embodiment of the invention.
Figure 2B is a partially exploded detailed view of a portion of the thermal pad of Figure 2A, according to one embodiment of the invention.
Figure 2C is an exploded view of a temperature control circuit board, according to one embodiment of the invention.
Figure 3 is a flowchart of a method to control the temperature of the thermal pad, according to one embodiment of the invention.

### DETAILED DESCRIPTION OF THE INVENTION

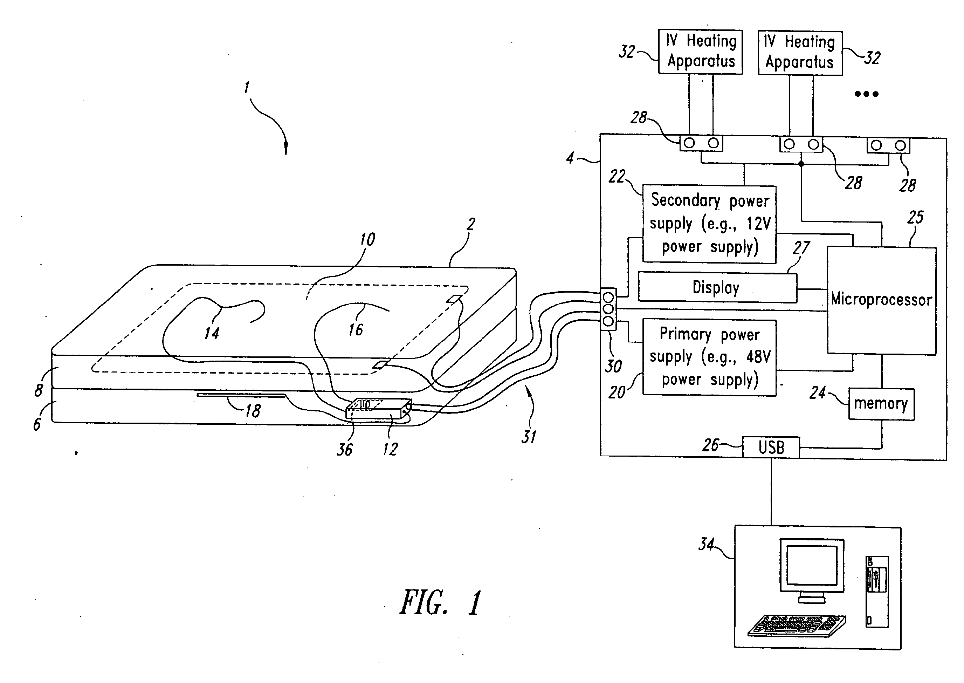

Figure 1 shows a schematic illustration of a temperature management system 1 for a thermal pad 2, according to one embodiment of the invention.

The temperature management system 1 comprises the thermal pad 2 electrically coupled to a power unit 4. The thermal pad 2 includes a lower and an upper foam layer 6, 8, having a heating element 10 sandwiched therebetween. A temperature control circuit board 12 is embedded within the lower foam layer 6 and positioned proximate a corner of the thermal pad 2. The temperature control circuit board 12 is positioned in the corner of the thermal pad 2 or otherwise advantageously positioned within the thermal pad 2 such that it is outside a range of radiation (*e.g*., X-ray) and an expected patient position when they are reclining on the pad. First and second surface temperature sensors 14, 16 are positioned within the upper foam layer 8 and proximate the surface of the thermal pad 2. The first and second surface sensors 14, 16 may be arranged such that the surface temperature of the thermal pad 2 at different locations may be determined. Additionally, a heating element sensor 18 is embedded within the lower foam layer 6 and proximate the heating element 10. The heating element sensor 18 is operable to determine the current temperature of the heating element 10, while the first and second surface sensors 14, 16 are operable to determine the temperature at several locations at the surface of the thermal pad 2. The temperature control circuit board 12 does not allow the temperature at the thermal pad 4 surface to exceed a maximum safe temperature (*e.g.*, approximately 40°C) and keeps it within a selected temperature range when in operation. In one embodiment, the heating element 10 may be heated to a maximum safe temperature.

The power device 4 includes a primary power supply 20, a secondary power supply 22, a memory board 24, a microprocessor 25, a USB port 26, a display 27, a plurality of IV fluid ports 28, and a communications port 30. The microprocessor 25 provides electronic control of all the components in the power device 4 and the pad circuit board 12. The communications port 30 provides a data communications path 31 between the power device 4 and the thermal pad 2. The communications port 31 may take the form of a multi-pin design. The IV data ports 28 are respectively coupled to a plurality of IV heating apparatuses 32. The plurality of IV heating apparatuses 32 are powered by the secondary power supply 22. Each of the plurality of IV heating apparatuses 32 may comprise the various embodiments as described in U.S. Patent Application No.(Attorney docket number 600071.401). The USB port 26 may be coupled to a computer system 34 to allow for transfer of stored data in the memory 24 to the computer system 34. The USB port 26 allows access to the memory 24 and various software programs of the computer system 34 (*e.g.*, an Excel spreadsheet). The computer system 34 may take the form of a PC, laptop or a hand-held device such as, for example cellular phone, blackberry, or the like. The computer system 34 may allow a wireless transmission of data to and from the power unit 4.

The secondary power supply 22 may take the form of a 12-volt power supply operable to power the power unit 4, each of the plurality of IV heating apparatuses 32, and the temperature control circuit board 12. The primary power supply 20 may take the form of a 48-volt power supply operable to power the heating element 10 embedded within the thermal pad 2. The 12-volt and 48-volt power supplies may, for example, be supplied by Condor Electronics and are useful for the conversion of power from AC to DC power. The data communications path 31 may be one of wireless or wired communications path. When the data communications path 31 takes the form of a wired communications path, the wire may be a bidirectional cable. In other words, the wire is removable at each end. The bidirectional cable may advantageously allow for the cable to be replaced without having to replace the power unit 4 coupled thereto.

A user may select an amount of power to be transferred from the primary power supply to the heating element 10, such that the heating element 10 may heat the surface of the thermal pad 2 to a desired temperature within a safe temperature range (*e.g*., less than 40°C). The user or the system software in the microprocessor 25 may select of range of temperatures within which to maintain the temperature of the pad, depending on the desired use and operating room procedures. For example, the pad may maintain a temperature in the range of 30-38°C, or within some other selected range. The amount of power supplied to the heating element 10 substantially predicts the amount of heat transferred to the surface of the pad 4, when no patient is positioned thereon.

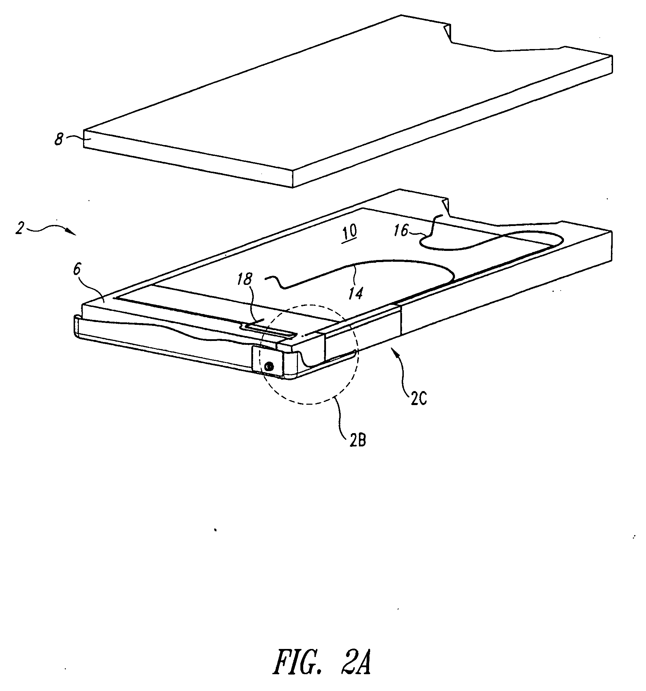

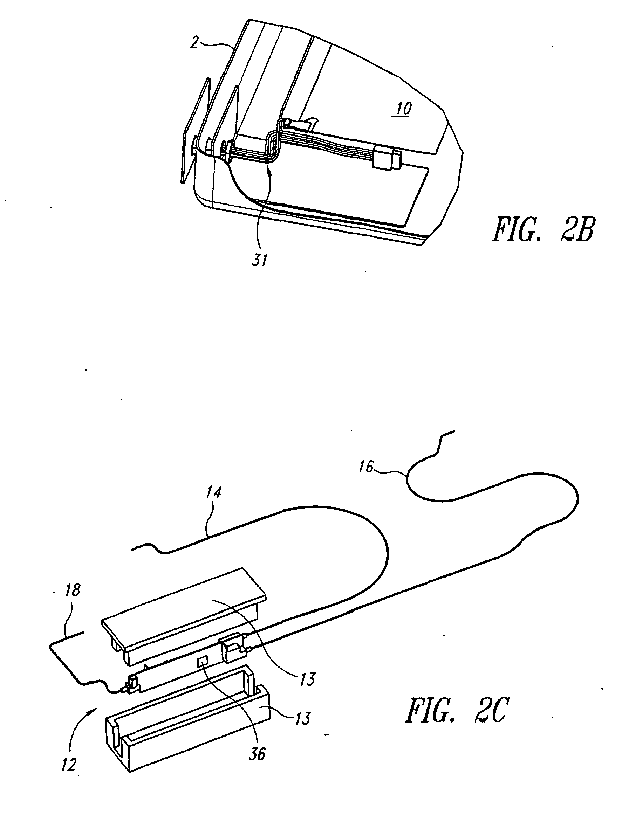

Figure 2A shows an isometric exploded view of the thermal pad 2 and the temperature control circuit board 12 embedded therein, according to one embodiment of the invention. Figure 2B shows a partially exploded detailed view of a portion of the thermal pad 2, while Figure 2C shows an exploded view of the temperature control circuit board 12, according to one embodiment of the invention.

The temperature control circuit board 12 may take the form of a three-beam splinter design. The temperature control circuit board 12 may be a fiber optic circuit board encased within an antistatic foam encasement 13. The antistatic foam encasement 13 may prevent electrical discharge. The first and second surface temperature sensors 14, 16, as well as the heating element temperature sensor 18, may, for example, be fiber optic or infrared sensors coupled to the circuit board 12. Having the circuit board 12 and the sensors 14, 16, 18 embedded within the thermal pad 2, eliminates the need for an external fiber optic cable coupling the sensors 14, 16, 18 inside the thermal pad 2 to the power board 4 that is outside the pad, rather, the circuit board 12 is electrical coupled by the bus of wires 31 to a location outside the thermal pad 2.

It is advantageous for the fiber optic cables to be embedded within the thermal pad 2 such that less expensive bi-directional cables, which are easily replaceable, may be used to form the communication path 31.

The temperature control circuit board 12 includes a microprocessor 36. The microprocessor 36 is operable to receive temperature data from the first and second surface sensors 14, 16 as well as the heating element sensor 18. The heating element 10 is controlled based on temperature information received from at least one of the surface sensors 14, 16 or the heating element sensor 18. The microprocessor 36 receives temperature data from the surface sensors 14, 16 and the heating element sensor 18, and controls the heating element 10 according to temperature data received from at least one of the surface sensors 14, 16 or the heating element sensor 18. At any given time, the microprocessor 36 selects the one of the specific sensors for which control of the heating element 10 is based.

When initially activated, the power unit 4 powers the heating element 10 to a default temperature of, for example, approximately 37°C. The surface temperature of the thermal pad 4 may also be at the default temperature (e.g., 37°C), if no patient is present on the pad 4. Otherwise, if a patient is present on the thermal pad 4, the patient may cause a thermodynamic transfer of heat from a portion of the patient's body to the thermal pad 4. The amount of heat added to the thermal pad 4 may vary based on a size, shape and weight of the patient.

The microprocessor 36 is operable to determine whether a patient is present on the thermal pad 4. For example, the microprocessor 36 may compare the temperature data from the first and second surface temperature sensors 14, 16 to temperature data from the heating element sensor 18, or compare the temperature data of the first and second surface sensors 14, 16 to a temperature of, for example, approximately 32°C. When at least one of the first and second sensors 14, 16 detects a temperature that is greater than the detected temperature of the heating element sensor 18 or greater than approximately 32°C, the patient is present on the pad 4. In response, the power unit 4 may activate an audible alarm to indicate such an over-temperature event. The audible alarm may, for example, be manufactured by Floyd Bell, Inc. and take the form of a chime, blare or ringing. Once it is determined that the patient is present thereon and causes additional heating of the thermal pad 2, the microprocessor 36 selects temperature data from the first and second surface sensors 14, 16 to control the heating element 10. Such guarantees that the surface temperature of the thermal pad 4 does not exceed a safe temperature. Otherwise, if it is determined that no patient is present on the thermal pad 4, the microprocessor 36 selects temperature data from the heating element sensor 18 to control the heating element 10.

In other words, when the patient is present on the thermal pad 4 and causes additional heating of the thermal pad 4, the first and second surface sensors 14, 16 may serve as master sensors while the heating element sensor 18 serves as a slave sensor. When no patient is present thereon, the heating element sensor 18 may be the master sensor while the first and second surface sensors 14, 16 serve as the slave sensors. The temperature data from the master sensor(s) is selected by the microprocessor 36 to control the heating element 10 rather than the temperature data of the slave sensor(s).

The microprocessor 36 controls the heating element 10 by instructing the power unit 4 to supply an amount of power to the heating element 10 that enables the heating element 10 to maintain a desired temperature. The amount of power supplied to the heating element 10 depends on the temperature data from the master sensor(s) 14, 16, 18.

In some embodiments the patient may fully recline along the length of the thermal pad 4 while in other embodiments the patient may partially recline or otherwise sit on a portion of the surface of the thermal pad 4. According to one embodiment, the patient may sit on the portion of the thermal pad 4 while having one of the first and the second surface sensors 14, 16 thereunder. Thus, according to such embodiment, one of the surface sensors 14, 16 serves as the master sensor while the other sensor 14 or 16 and the heating element sensor 18 serve as the slave sensors. For example, if the first surface sensor 14 is positioned underneath the patient while the second surface sensor 16 is not, there will be a thermodynamic transfer of heat from the patient to the thermal pad 4 surface proximate the first surface sensor 14. Thus, in such example, the first surface sensor 14 would be the master sensor while the second surface sensor 16 and the heating element sensor 18 would be the slave sensor. The microprocessor 36 may use the detected temperature from the first surface sensor 14 to control the heating element 10.

According to one embodiment, a user may select a desired temperature of the thermal pad 4 (e.g., greater than 37C). Although the heating element sensor 18 detects a temperature value substantially equivalent to a selected temperature, the first and second surface sensors 14, 16 may detect a temperature value greater than the selected temperature (i.e., in response to a patient reclining thereon and thermodynamically producing additional heat). In response, the temperature control circuit board 12 transmits temperature data of the first and second surface sensors 14, 16 to the power unit 4 to cause the power provided to the heating element 10 to be reduced or otherwise deactivated to reduce the surface temperature of the thermal pad 4 to a safe temperature. When the first and second surface sensors 14, 16 detect a temperature value greater than a temperature detected by the heating element sensor 18, the first and second surface sensors 14, 16 become the master sensors, while the heating element sensor 18 becomes a slave sensor. When the heating element sensor 18 detects a higher temperature value than the first and second surface sensors 14, 16, the heating element sensor 18 becomes the master sensor while the first and second surface sensors 14, 16 become the slave sensors. The determination of the master and the slave sensor prevents the thermal pad 4 from exceeding the selected temperature in response to various size, shape, and weight of patients. Such determination by the microprocessor 36 is advantageous in that it prevents the pad 4 surface from overheating a patient that may be disposed thereon. Overheating based on localized temperature sensing that is not representative of the actual patient temperature is therefore avoided.

The microprocessor 36 also determines errors in the sensors and transmits an indication of such errors to the power unit 4. The power unit 4 may serve as a self-diagnosing device within the processor 25 that may indicate malfunctions, such as, for example, whether a specific sensor has been disconnected. Malfunctions or errors in warming the thermal pad 2 and/or the IV heating apparatuses 32 may be exhibited in the form of LEDs having various colors. For example, a green LED may suggest proper thermal operation while a red LED may suggest a malfunction or an error in the heating of the IV fluid or the pad 2.

The power unit 4 maintains consistent control of the temperature of the patient from multiple points of operation. It control the temperature of the IV fluid being input to the patient and can warm the fluid to a slightly higher temperature if the body of the patient is deemed to cool. The insertion of warmer fluid is a more direct and rapid way to directly warm internal organs and the internal body functions than merely the surface heaters from the pad 10. The microprocessor 25 can coordinate and control a uniform temperature management system for the patient for multiple heating sources, one the thermal pad on which the patient reclines and the other an IV fluid input directly to the patient's blood vessels.

In one embodiment, the IV fluid heaters 32 and the ports 28 are not present, and in such embodiments, the temperature of the patient is controlled from the pad 10.

The power unit 4 coupled to the thermal pad 2 is a multi-functional unit that provides controlled and safe application system for warming a patient positioned on the thermal pad 2 (e.g., operating room table) and, in one embodiment, also for warming IV fluids. The power unit 4 may be used during surgery to ensure that the patient remains warmed at a safe temperature and within a selected temperature range. According to one embodiment, the power unit may be 7" x 7" x 9"-7" (i.e., inclined operational face) and is easily clamped to an IV pole or other tubular mounting surface. The power unit 4 may, for example, be clamped to the IV pole with a GCX PC1000 clamp. The power unit 4 may include mounting pads for positioning on a surface, such as, for example, the operating room (OR) table pedestal.

The power unit 4 includes an operator control display 27 that allows for easy visual monitoring or manipulation when mounted to a lower portion of the IV pole. A single encasement of the power unit 4 allows for ease of fluid shedding and cleaning. The single encasement creates an internal cavity which allows sufficient air circulation to maintain a cool operating environment.

The memory board 24 embedded within the power unit 4 is operable to store at least 100 hours of operating history of the thermal pad 4 and operating history of the IV heating apparatuses 32. The operating history may be transferred through the USB port 26 to a spreadsheet (e.g., an Excel spreadsheet) in the computing system 34. Alternatively, the operating history may be wirelessly transmitted to and from the computing system 34 over a secure network such as, for example a WAN or LAN.

An operator of the power unit 4 may access applications in the computer system 34 via the USB port 26 and transfer data therebetween. For example, the operator may access the operating history of both the thermal pad 2 and the IV heating apparatus 32 for a specific patient over a defined period of time. In one embodiment, the power unit 4 has a clock and calendar feature that allows the operator to set the local date and time. The operator may set a timer such that the power unit 4 is activated after a defined amount of time. Additionally and/or alternatively, the power unit 4 may be set to deactivate after a desired amount of time.

Furthermore, the power unit 4 is operable to display data in at least one of several languages. Such allows for the power unit 4 to be programmable to languages used by medical practitioners around the world. The programmable features of the power unit 4 may be accessed via an accompanying computer readable medium (e.g., floppy disk, compact disk, a USB drive, etc.).

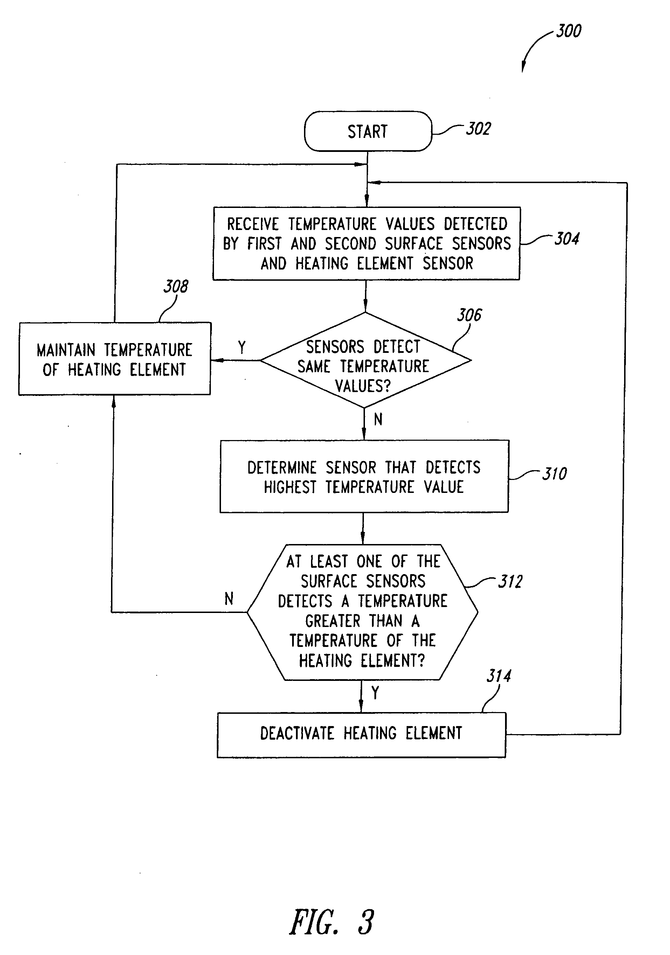

Figure 3 shows a flow diagram of a method 300 of controlling a temperature management system of the thermal pad 2, according to one embodiment of the invention.

The method 300 starts at 302, for example in response to an activation of the power unit 4. The power unit 4 may be activated manually (i.e., user activated) or automatically (i.e., timer activated). As mentioned above, the power unit 4 may be initially activated to cause the heating element 10 to heat to a default temperature value of, for example, approximately 37°C. Alternatively, the user may select the amount of power to be transferred from the primary power supply to the heating element 10, such that the heating element 10 may heat the surface of the thermal pad 2 to the desired temperature. The desired and the default temperatures are in the safe temperature range (*e.g*., less than 40° C).

At 304, the temperature control circuit board 12 receives temperature values respectively detected by the first surface sensor 14, the second surface sensor 16 and the heating element sensor 18.

At 306, the microprocessor 36 determines whether the first surface sensor 14, the second surface sensor 16 and the heating element sensor 18 detect substantially same temperature values. In response to a determination that the first surface sensor 14, the second surface sensor 16 and the heating element sensor 18 detect substantially same temperature values control passes to 308.

At 308, the microprocessor 36 instructs the power unit 4 to maintain the supply of power to the heating element 10. The supply of power to the heating element 10 allows for the thermal pad 2 to remain at the desired temperature. The method passes control to 304.

At 310, in response to a determination that the first surface sensor 14, the second surface sensor 16 and the heating element sensor 18 detect substantially different temperature values, the microprocessor 36 determines which of the sensors 14, 16, 18 detects the highest temperature value. The microprocessor will use the temperature from the surface temperature sensor that detects the highest value on which to based the temperature control. Thus, if the surface temperature sensor 14 shows a higher temperature than surface temperature sensor 16, then 14 will become the master on which control is based. Once either of the surface temperature sensors is above a selected value, for example 32°C in one case, or a another selected temperature in another case, they will become the masters for controlling the temperature since they more accurately reflect the true temperate of the patient.

At 312, the microprocessor 36 determines whether at least one of the surface sensors 14, 16 detects a temperature greater than a temperature of the heating element 10. At 314, in response to a determination that at least one of the surface sensors 14, 16 detects a temperature value that is greater than a value determined by the heating element sensor 18, the microprocessor 36 instructs the power unit 4 to deactivate or otherwise reduce the supply of power to the heating element 10. The power unit 4 may be deactivated until the temperature at the surface of the thermal pad 2 is substantially equivalent to the temperature of the heating element 10 or within the safe temperature range. Alternatively, reducing the power supplied to the heating element 10 causes the heating element 10 to dissipate less heat, thereby reducing the temperature of the heating element 10 and the temperature at the surface of the thermal pad 2. The method passes control to 304.

Otherwise, if the heating element sensor 18 detects a greater temperature value than the first and second surface sensors 14, 16, the method passes control to 308 to maintain the proper temperature.

From the foregoing it will be appreciated that, although specific embodiments of the invention have been described herein for purposes of illustration, various modifications may be made without deviating from the scope of the invention. In this description, certain specific details are set forth in order to provide a thorough understanding of various embodiments of the invention. One skilled in the art will understand that the embodiments may be practiced without these details. Of course well-known structures, equipment and processes associated fluid warming devices, including power sources and resulting structures have not been shown or described in detail to avoid unnecessarily obscuring the description. Furthermore, the particular features, structures, or characteristics may be combinable in any suitable manner in one or more embodiments. Accordingly, the invention is not limited except as by the appended claims.

## Claims

1. An apparatus for warming a patient on a thermal pad (2) the apparatus comprising:
a heating element (10) adapted to heat a surface of the thermal pad (2),
a power unit (4) operable to provide power to the heating element (10);
a heating element temperature sensor (18) adapted to detect a temperature of the heating element (10);
a plurality of surface temperature sensors (14, 16) adapted to detect temperature at a plurality of select portions of the surface of the thermal pad (2); **characterized in that** the apparatus comprises
a temperature control circuit board (12) electrically coupled to each of the temperature sensors (14, 16, 18), the temperature control circuit board (12) operable to maintain a temperature at the surface of the thermal pad (2) within a selected temperature range based on the temperature detected by at least one of the temperature sensors (14, 16, 18), and **characterised in that** said temperature control circuit board is embedded within the thermal pad (2).

2. The apparatus of claim 1 wherein the temperature control circuit board (12) comprises a microprocessor (36) to receive the temperature detected by the plurality of temperature sensors (14, 16, 18), and wherein the microprocessor (36) determines that a patient is present on the thermal pad when the temperature detected by at least one of the plurality of surface temperature sensors (14, 16) is above 32°C.

3. The apparatus of claim 1 wherein the temperature control circuit board (12) comprising a microprocessor (36) to receive the temperature detected by the plurality of temperature sensors (14, 16, 18), and wherein the microprocessor (36) determines if the patient is present on the thermal pad (2) when the temperature detected by at least one of the plurality of surface temperature sensors (14, 16) is substantially greater than the temperature detected by the heating element sensor (18).

4. The apparatus of claim 1 wherein the temperature control circuit board (12) comprising a microprocessor (36) to receive the temperature detected by the plurality of temperature sensors (14, 16, 18), and wherein the microprocessor (36) selects the temperature detected by at least one of the plurality of surface temperature sensors (14, 16) to control the heating element (10) in response to the temperature detected by at least one of the plurality of surface temperature sensors (14, 16) being substantially greater than the temperature detected by the heating element sensor (18).

5. The apparatus of claim 4 wherein the temperature control circuit board (12) transmits the temperature detected by the at least one of the plurality of surface temperature sensors (14, 16) to the power unit (4) to cause the power provided to the heating element (10) to be reduced such that the amount of heat being provided to the thermal pad (2) is reduced.

6. The apparatus of claim 1 wherein the temperature control circuit board (12) comprising a microprocessor (36) to receive the temperature detected by the plurality of temperature sensors (14, 16, 18), and wherein the microprocessor (36) selects the temperature detected by the heating element temperature sensor (18) to control the heating element (10) in response to the temperature detected by at least one of the plurality of sensors (14, 16) being substantially equal to or less than the temperature detected by the heating element sensor (18).

7. The apparatus of claim 6 wherein the temperature control circuit board (12) transmits the temperature detected by the heating element temperature sensor (18) to the power unit (4) to cause the power provided to the heating element (10) to be maintained such that the temperature of the heating element (10) is unchanged.

8. The apparatus of claim 1 wherein the power unit (4) comprises a memory operable to store at least 100 hours of operating data including the temperature detected by the plurality of surface temperature sensors (14, 16) and the heating element temperature sensor (18), and the power applied to the heating element (10).

9. The apparatus of claim 8 wherein the power unit is further operable to power at least one intravenous (IV) fluid heating apparatus and store at least 100 hours of fluid heating data in the memory.

10. The apparatus of claim 9 wherein the memory is operable to transmit the operating data and the fluid heating data to a computer system along a data communications path.

11. The apparatus of claim 10 wherein the data communications path is a wireless communications path and the computer system takes the form of a hand-held device capable of wirelessly communicating with the power unit.

12. The apparatus of claim 1 wherein the temperature control circuit board (12) is a fiber optic board encased within an antistatic foam encasement.

13. The apparatus of claim 1 wherein the plurality of sensors (14, 16) and the heating element sensor (18) are at least one of fiber optic and infrared sensors.

14. A method for warming a patient on a thermal pad using the apparatus of claim 1, the method comprising:
sensing the temperature of a heating element (10) within a central portion of the thermal pad (2);
wherein the method comprises:
sensing the temperature of a plurality of surface temperature sensors (14, 16) positioned at selected portions of the surface of the thermal pad (2) to detect temperature at the surface of the thermal pad (2); and
regulating the power provided to the heating element (10) based on the temperature detected by at least one of the plurality of surface temperature sensors (14, 16) and a heating element temperature sensor (18) to maintain a temperature at the surface of the thermal pad (2) within a selected temperature range.

15. The method of claim 14, further comprises determining a presence of the patient on the thermal pad (2) in response to the temperature detected by at least one of the plurality of surface temperature sensors (14, 16) being above 32°C.

16. The method of claim 14, further comprises determining a presence of the patient on the thermal pad (2) in response to the temperature detected by at least one of the plurality of surface temperature sensors (14, 16) being substantially greater than the temperature detected by the heating element sensor (18).

17. The method of claim 16 wherein determining the presence of the patient on the thermal pad (2) includes transmitting the temperature detected by at least one of the plurality of surface temperature sensors (14, 16) to the power unit (4) to cause the power provided to the heating element (10) to be reduced or otherwise deactivated to lower the temperature at the surface of the thermal pad (2) to the safe temperature.

18. The method of claim 16 wherein determining the presence of the patient on the thermal pad (2) includes transmitting the temperature detected by the heating element sensor (18) to the power unit (4) to cause the power provided to the heating element (10) to be maintained such that the temperature of the heating element (10) is unchanged.

## Patentansprüche

1. Vorrichtung zum Erwärmen eines Patienten auf einer Wärmeunterlage (2), wobei die Vorrichtung Folgendes umfasst:
ein Heizelement (10), das zum Beheizen einer Oberfläche der Wärmeunterlage (2) geeignet ist;
eine Stromversorgungseinheit (4), die betreibbar ist, um das Heizelement (10) mit Strom zu versorgen;
einen Heizelementtemperatursensor (18), der zum Ermitteln einer Temperatur des Heizelements (10) geeignet ist;
mehrere Oberflächentemperatursensoren (14, 16), die zum Ermitteln einer Temperatur an mehreren der ausgewählten Teile der Oberfläche der Wärmeunterlage (2) geeignet sind; **dadurch gekennzeichnet, dass** die Vorrichtung eine Temperatursteuerungsplatine (12) umfasst, die elektrisch mit jedem der Temperatursensoren (14, 16, 18) verbunden ist, wobei die Temperatursteuerungsplatine (12) betreibbar ist, um eine Temperatur an der Oberfläche der Wärmeunterlage (2) innerhalb eines ausgewählten Temperaturbereichs basierend auf der Temperatur beizubehalten, die von mindestens einem der Temperatursensoren (14, 16, 18) ermittelt wird, und **dadurch gekennzeichnet, dass** die Temperatursteuerungsplatine in der Wärmeunterlage (2) eingebettet ist.

2. Vorrichtung nach Anspruch 1, wobei die Temperatursteuerungsplatine (12) einen Mikroprozessor (36) zum Empfangen der Temperatur umfasst, die von den mehreren Temperatursensoren (14, 16, 18) ermittelt wird, und wobei der Mikroprozessor (36) bestimmt, dass sich ein Patient auf der Wärmeunterlage befindet, wenn die von mindestens einem der mehreren Oberflächentemperatursensoren (14, 16) ermittelte Temperatur über 32°C ist.

3. Vorrichtung nach Anspruch 1, wobei die Temperatursteuerungsplatine (12) einen Mikroprozessor (36) zum Empfangen der Temperatur umfasst, die von den mehreren Temperatursensoren (14, 16, 18) ermittelt wird, und wobei der Mikroprozessor (36) bestimmt, ob sich der Patient auf der Wärmeunterlage (2) befindet, wenn die von mindestens einem der mehreren Oberflächentemperatursensoren (14, 16) ermittelte Temperatur im Wesentlichen höher als die vom Heizelementsensor (18) ermittelte Temperatur ist.

4. Vorrichtung nach Anspruch 1, wobei die Temperatursteuerungsplatine (12) einen Mikroprozessor (36) zum Empfangen der von den mehreren Temperatursensoren (14, 16, 18) ermittelten Temperatur umfasst, und wobei der Mikroprozessor (36) die von mindestens einem der mehreren Oberflächentemperatursensoren (14, 16) ermittelte Temperatur auswählt, um das Heizelement (10) in Reaktion auf die von mindestens einem der mehreren Oberflächentemperatursensoren (14, 16) ermittelte Temperatur zu steuern, damit sie im Wesentlichen höher als die von dem Heizelementsensor (18) ermittelte Temperatur ist.

5. Vorrichtung nach Anspruch 4, wobei die Temperatursteuerungsplatine (12) die von mindestens einem der mehreren Oberflächentemperatursensoren (14, 16) ermittelte Temperatur an die Stromversorgungseinheit (4) überträgt, damit der dem Heizelement (10) bereitgestellte Strom reduziert wird, damit die für die Wärmeunterlage (2) bereitgestellte Wärme reduziert wird.

6. Vorrichtung nach Anspruch 1, wobei die Temperatursteuerungsplatine (12) einen Mikroprozessor (36) zum Empfangen der von den mehreren Temperatursensoren (14, 16, 18) ermittelten Temperatur umfasst, und wobei der Mikroprozessor (36) die vom Heizelementtemperatursensor (18) ermittelte Temperatur auswählt, um das Heizelement (10) in Reaktion auf die von mindestens einem der mehreren Sensoren (14, 16) ermittelte Temperatur zu steuern, damit sie im Wesentlichen gleich oder niedriger als die von dem Heizelementsensor (18) ermittelte Temperatur ist.

7. Vorrichtung nach Anspruch 6, wobei die Temperatursteuerungsplatine (12) die vom Heizelementtemperatursensor (18) ermittelte Temperatur an die Stromversorgungseinheit (4) überträgt, damit der für das Heizelement (10) bereitgestellte Strom so beibehalten wird, dass die Temperatur des Heizelements (10) unverändert bleibt.

8. Vorrichtung nach Anspruch 1, wobei die Stromversorgungseinheit (4) einen Speicher umfasst, der betreibbar ist, um mindestens 100 Stunden Betriebsdaten, einschließlich der Temperatur, die von den mehreren Oberflächentemperatursensoren (14, 16) und dem Heizelementtemperatursensor (18) ermittelt wird, und des Stroms, der an das Heizelement (10) angelegt wird, zu speichern.

9. Vorrichtung nach Anspruch 8, wobei die Stromversorgungseinheit ferner betreibbar ist, um mindestens eine intravenöse (IV) Flüssigkeitsheizvorrichtung mit Strom zu versorgen, und um mindestens 100 Stunden Flüssigkeitsheizdaten im Speicher zu speichern.

10. Vorrichtung nach Anspruch 9, wobei der Speicher betreibbar ist, um die Betriebsdaten und die Flüssigkeitsheizdaten entlang einem Datenkommunikationspfad an ein Computersystem zu übertragen.

11. Vorrichtung nach Anspruch 10, wobei der Datenkommunikationspfad ein drahtloser Kommunikationspfad ist, und das Computersystem die Form einer tragbaren Vorrichtung annimmt, die drahtlos mit der Stromversorgungseinheit kommunizieren kann.

12. Vorrichtung nach Anspruch 1, wobei die Temperatursteuerungsplatine (12) eine Faseroptikplatte ist, die von einer antistatischen Schaumstoffumhüllung eingeschlossen ist.

13. Vorrichtung nach Anspruch 1, wobei die mehreren Sensoren (14, 16) und der Heizelementsensor (18) ein Faseroptik- oder ein Infrarotsensor sind.

14. Verfahren zum Erwärmen eines Patienten auf einer Wärmeunterlage mithilfe der Vorrichtung gemäß Anspruch 1, wobei das Verfahren Folgendes umfasst:
Messen der Temperatur eines Heizelements (10) innerhalb eines zentralen Teils der Wärmeunterlage (2);
wobei das Verfahren Folgendes umfasst:
Messen der Temperatur von mehreren Oberflächentemperatursensoren (14, 16), die an ausgewählten Teilen der Oberfläche der Wärmeunterlage (2) positioniert sind, um die Temperatur auf der Oberfläche der Wärmeunterlage (2) zu ermitteln; und
Regulieren des dem Heizelement (10) bereitgestellten Stroms basierend auf der Temperatur, die von mindestens einem der mehreren Oberflächentemperatursensoren (14, 16) und einem Heizelementtemperatursensor (18) ermittelt werden, um eine Temperatur an der Oberfläche der Wärmeunterlage (2) innerhalb einem ausgewählten Temperaturbereich beizubehalten.

15. Verfahren nach Anspruch 14, ferner umfassend das Bestimmen des Vorhandenseins des Patienten auf der Wärmeunterlage (2) in Reaktion darauf, dass die von mindestens einem der mehreren Oberflächentemperatursensoren (14, 16) ermittelte Temperatur über 32°C ist.

16. Verfahren nach Anspruch 14, ferner umfassend das Bestimmen des Vorhandenseins des Patienten auf der Wärmeunterlage (2) in Reaktion darauf, dass die von mindestens einem der mehreren Oberflächentemperatursensoren (14, 16) ermittelte Temperatur im Wesentlichen höher als die von dem Heizelementsensor (18) ermittelte Temperatur ist.

17. Verfahren nach Anspruch 16, wobei das Bestimmen des Vorhandenseins des Patienten auf der Wärmeunterlage (2) das Übertragen der von mindestens einem der mehreren Oberflächentemperatursensoren (14, 16) ermittelten Temperatur an die Stromversorgungseinheit (4) umfasst, damit der dem Heizelement (10) zur Verfügung gestellte Strom reduziert oder andernfalls deaktiviert wird, um die Temperatur auf der Oberfläche der Wärmeunterlage (2) auf die sichere Temperatur zu senken.

18. Verfahren nach Anspruch 16, wobei das Bestimmen des Vorhandenseins des Patienten auf der Wärmeunterlage (2) das Übertragen der vom Heizelementtemperatursensor (18) ermittelten Temperatur an die Stromversorgungseinheit (4) umfasst, damit der dem Heizelement (10) bereitgestellte Strom so beibehalten wird, dass die Temperatur des Heizelements (10) unverändert bleibt.

## Revendications

1. Dispositif pour réchauffer un patient sur un matelas thermique (2), le dispositif comportant :
un élément chauffant (10) conçu pour chauffer une surface du matelas thermique (2) ;
une unité d'alimentation électrique (4) pouvant agir pour fournir de la puissance à l'élément chauffant (10);
un capteur (18) de température de l'élément chauffant conçu pour détecter la température de l'élément chauffant (10) ;
une pluralité de capteurs de température de surface (14, 16) adaptés pour détecter la température au niveau d'une pluralité de parties sélectionnées de la surface du matelas thermique (2), **caractérisé en ce que** le dispositif comporte
une carte de circuit (12) pour la commande de température couplée électriquement à chacun des capteurs de température (14, 16, 18), la carte de circuit (12) pour la commande de température (12) pouvant agir pour maintenir une température au niveau de la surface du matelas thermique (2) dans une plage de températures sélectionnée sur la base de la température détectée par au moins l'un des capteurs de température (14, 16, 18), et **caractérisé en ce que** ladite carte de circuit pour la commande de température est logée à l'intérieur du matelas thermique (2).

2. Dispositif selon la revendication 1 dans lequel la carte de circuit (12) pour la commande de température comprend un microprocesseur (36) pour recevoir la température détectée par la pluralité des capteurs de température (14, 16, 18), et dans lequel le microprocesseur (36) détermine qu'un patient est présent sur le matelas thermique (2) lorsque la température détectée par au moins l'un de la pluralité des capteurs de température de surface (14, 16) est supérieure à 32°C.

3. Dispositif selon la revendication 1 dans lequel la carte de circuit (12) pour la commande de température comporte un microprocesseur (36) pour recevoir la température détectée par la pluralité de capteurs de température (14, 16, 18) et dans lequel le microprocesseur (36) détermine si le patient est présent sur le matelas thermique (2) lorsque la température détectée par au moins l'un de la pluralité des capteurs de température de surface (14, 16) est essentiellement supérieure à la température détectée par le capteur de l'élément chauffant (18).

4. Dispositif selon la revendication 1 dans lequel la carte de circuit (12) pour la commande de température comporte un microprocesseur (36) pour recevoir la température détectée par la pluralité de capteurs de température (14, 16, 18) et dans lequel le microprocesseur (36) sélectionne la température détectée par au moins un capteur de la pluralité des capteurs de température de surface (14, 16) afin de commander l'élément chauffant (10) en réponse à la température détectée par au moins l'un de la pluralité des capteurs de température de surface (14, 16) qui est essentiellement plus élevée que la température détectée par le capteur de l'élément chauffant (18).

5. Dispositif selon la revendication 4 dans lequel la carte de circuit (12) pour la commande de température transmet la température détectée par au moins l'un de la pluralité de capteurs de la température de surface (14, 16) à l'unité d'alimentation électrique (4) pour entraîner la réduction de la puissance fournie à l'élément chauffant (10) de telle façon que la quantité de chaleur fournie au matelas thermique (2) soit réduite.

6. Dispositif selon la revendication 1 dans lequel la carte de circuit (12) pour la commande de température comporte un microprocesseur (36) pour recevoir la température détectée par la pluralité de capteurs de température (14, 16, 18) et dans lequel le microprocesseur (36) sélectionne la température détectée par le capteur de température (18) de l'élément chauffant afin de commander l'élément chauffant (10) en réponse à la température détectée par au moins l'un de la pluralité de capteurs (14, 16) qui est sensiblement égale, ou inférieure, à la température détectée par le capteur de l'élément chauffant (18).

7. Dispositif selon la revendication 6 dans lequel la carte de circuit (12) pour la commande de température transmet la température détectée par le capteur de température (18) de l'élément chauffant à l'unité d'alimentation électrique (4) pour entrainer le maintien de la puissance fournie à l'élément chauffant (10) de telle sorte que la température de l'élément chauffant (10) reste inchangée.

8. Dispositif selon la revendication 1 dans lequel l'unité d'alimentation électrique (4) comporte une mémoire opérationnelle pour stocker au moins 100 heures de données de fonctionnement comprenant la température détectée par la pluralité des capteurs de température de surface (14, 16) et le capteur de température (18) de l'élément chauffant, et la puissance appliquée à l'élément chauffant (10).

9. Dispositif selon la revendication 8 dans lequel l'unité d'alimentation électrique peut agir, de plus, pour alimenter électriquement au moins un appareil de chauffage de fluide intraveineux (IV) et stocker dans la mémoire au moins 100 heures de données de chauffage de fluide.

10. Dispositif selon la revendication 9 dans lequel la mémoire est opérationnelle pour transmettre les données de fonctionnement et les données de chauffage de fluide à un système informatique le long d'une voie de communications de données.

11. Dispositif selon la revendication 10 dans lequel la voie de communications de données est une voie de communications sans fil et dans lequel le système informatique prend la forme d'un dispositif tenu à la main capable de communiquer par une liaison sans fil avec l'unité d'alimentation électrique.

12. Dispositif selon la revendication 1 dans lequel la carte de circuit (12) pour la commande de température est une carte à fibre optique incorporée dans un enrobage de mousse antistatique.

13. Dispositif selon la revendication 1 dans lequel la pluralité des capteurs (14, 16) et le capteur de l'élément chauffant (18) sont au moins l'un des capteurs à fibre optique et à infrarouge.

14. Procédé pour réchauffer un patient sur un matelas thermique en utilisant le dispositif selon la revendication 1, le procédé comprenant le fait de :
détecter la température d'un élément chauffant (10) à l'intérieur d'une partie centrale du matelas thermique (2) ;
lequel procédé comprend le fait de :
détecter la température d'une pluralité de capteurs de température de surface (14, 16) positionnés au niveau de parties sélectionnées de la surface du matelas thermique (2) afin de détecter la température au niveau de la surface du matelas thermique (2) et
réguler la puissance fournie à l'élément chauffant (10) sur la base de la température détectée par au moins l'un de la pluralité des capteurs de température de surface (14, 16) et d'un capteur de température (18) de l'élément chauffant afin de maintenir une température au niveau de la surface du matelas thermique (2) à l'intérieur d'une plage de températures sélectionnée.

15. Procédé selon la revendication 14 comprenant, de plus, le fait de déterminer la présence d'un patient sur le matelas thermique (2) en réponse à la température détectée par au moins l'un de la pluralité des capteurs de température de surface (14, 16) qui est supérieure à 32°C.

16. Procédé selon la revendication 14 comprenant, de plus, le fait de déterminer la présence d'un patient sur le matelas thermique (2) en réponse à la température détectée par au moins l'un de la pluralité des capteurs de température de surface (14, 16) qui est essentiellement plus élevée que la température détectée par le capteur de l'élément chauffant (18).

17. Procédé selon la revendication 16 dans lequel la détermination de la présence du patient sur le matelas thermique (2) comporte le fait de transmettre la température détectée par au moins l'un de la pluralité des capteurs de température de surface (14, 16) à l'unité d'alimentation électrique (4) pour entraîner la réduction de la puissance fournie à l'élément chauffant (10) ou, sinon, la désactivation afin d'abaisser la température au niveau de la surface du matelas thermique (2) jusqu'à la température de sécurité.

18. Procédé selon la revendication 16 dans lequel la détermination de la présence du patient sur le matelas thermique (2) comporte la fait de transmettre la température détectée par le capteur de l'élément chauffant (18) à l'unité d'alimentation électrique (4) pour entraîner le maintien de la puissance fournie à l'élément chauffant (10) de telle sorte que la température de l'élément chauffant (10) reste inchangée.
